# EUROPEAN PATENT APPLICATION

(11) **EP 4 675 278 A2**
(43) Date of publication of application: **07.01.2026**
(21) Application number: 25217662.3
(22) Date of filing: 23.05.2018
(51) Int. Cl.: G01N 33/68

(54) **ASSAY FOR PLASMA CELL ASSOCIATED DISEASE**

(30) Priority: 23.05.2017 GB 201708262
(62) Divisional of application: 22175618.2
(71) Applicant: The Binding Site Group Limited, Birmingham, West Midlands B15 1QT (GB)
(72) Inventor: WALLIS, Gregg, Birmingham, B15 1QT (GB); HARDING, Stephen, Birmingham, B15 1QT (GB); HUGHES, Richard Geir, Birmingham, B15 1QT (GB)
(74) Representative: Henderson, Helen Lee

(57) **Abstract**

The application provides a method of identifying or monitoring a plasma cell associated disease, comprising purifying immunoglobulin free light chains (FLCs) from a sample from a subject with anti-FLC specific antibodies or fragments thereof and subjecting the purified sample to a mass spectrometry technique to identify the presence of one or more peaks corresponding to one or more monoclonal FLCs in the sample.

## Description

The invention relates to methods of identifying or monitoring plasma cell associated diseases by purifying free light chains (FLC) from a subject and detecting them using mass spectrometry.

Antibody molecules (also known as immunoglobulins) have a twofold symmetry and typically are composed of two identical heavy chains and two identical light chains, each containing variable and constant domains. The variable domains of the heavy and light chains combine to form an antigen-binding site, so that both chains contribute to the antigen-binding specificity of the antibody molecule. The basic tetrameric structure of antibodies comprises two heavy chains covalently linked by a disulphide bond. Each heavy chain is in turn attached to a light chain, again via a disulphide bond. This produces a substantially "Y"-shaped molecule.

Heavy chains are the larger of the two types of chain found in antibodies, with typical molecular mass of 50,000-77,000 Da, compared with the smaller light chain with a typical molecular mass of 22,000 to 25,000 Da.

There are five main classes or class or classes of heavy chain which are G, A, M, D and E which are the constituents heavy chains for: IgG, IgA, IgM, IgD and IgE respectively. IgG is the major immunoglobulin of normal human serum, accounting for 70-75% of the total immunoglobulin pool. This is the major antibody of secondary immune responses. It forms a single tetramer of two heavy chains plus two light chains.

IgM accounts for approximately 10% of the immunoglobulin pool. The molecules, together with J-chains, form a pentamer of five of the basic 4-chain structures. The individual heavy chains have a molecular weight of approximately 65,000 Da and the whole molecule has a molecular weight of about 970,000 Da. IgM is largely confined to the intravascular pool and is the predominant early antibody.

IgA represents 15-20% of human serum immunoglobulin pool. More than 80% of IgA occurs as a monomer. However, some of the IgA (secretory IgA) exists as a dimeric form.

IgD accounts for less than 1% of the total plasma immunoglobulin. IgD is found on the surface membrane of maturing B-cells.

IgE, although scarce in normal serum, is found on the surface membrane of basophils and mast-cells. It is associated with allergic diseases such as asthma and hay-fever.

In addition to the five main class or classes, there are four subclasses for IgG (IgG1, IgG2, IgG3 and IgG4). Additionally there are two subclasses for IgA (IgA1 and IgA2).

There are two types of light chain: Lambda (λ) and Kappa (κ). There are approximately twice as many κ as λ molecules produced in humans, but this is quite different in some mammals. Each chain contains approximately 220 amino acids in a single polypeptide chain that is folded into one constant and one variable domain. Plasma cells produce one of the five heavy chain types together with either κ or λ molecules. There is normally approximately 40% excess free light chain production over heavy chain synthesis. Where the light chain molecules are not bound to heavy chain molecules, they are known as "free light chain molecules". The κ light chains are usually found as monomers. The λ light chains tend to form dimers.

There are a number of proliferative diseases associated with antibody producing cells.

In many such proliferative diseases a plasma cell proliferates to form a monoclonal tumour of identical plasma cells. This results in production of large amounts of identical immunoglobulins and is known as a monoclonal gammopathy.

Diseases such as myeloma and primary systemic amyloidosis (AL amyloidosis) account for approximately 1.5% and 0.3% respectively of cancer deaths in the United Kingdom. Multiple myeloma is the second-most common form of haematological malignancy after non-Hodgkin lymphoma. In Caucasian populations the incidence is approximately 40 per million per year. Conventionally, the diagnosis of multiple myeloma is based on the presence of excess monoclonal plasma cells in the bone marrow, monoclonal immunoglobulins in the serum or urine and related organ or tissue impairment such as hypercalcaemia, renal insufficiency, anaemia or bone lesions. Normal plasma cell content of the bone marrow is about 1% of nucleated cells, while in multiple myeloma the content is typically greater than 10%, frequently greater than 30%, but may be over 90%.

AL amyloidosis is a protein conformation disorder characterised by the accumulation of monoclonal free light chain fragments as amyloid deposits. Typically, these patients present with heart or renal failure but peripheral nerves and other organs may also be involved.

There are a number of other diseases which can be identified by the presence of monoclonal immunoglobulins within the blood stream, or indeed urine, of a patient. These include plasmacytoma and extramedullary plasmacytoma, a plasma cell tumour that arises outside the bone marrow and can occur in any organ. When present, the monoclonal protein is typically IgA. Multiple solitary plasmacytomas may occur with or without evidence of multiple myeloma. Waldenström's macroglobulinaemia is a low-grade lymphoproliferative disorder that is associated with the production of monoclonal IgM. There are approximately 1,500 new cases per year in the USA and 300 in the UK. Serum IgM quantification is important for both diagnosis and monitoring. B-cell non-Hodgkin lymphomas cause approximately 2.6% of all cancer deaths in the UK and monoclonal immunoglobulins have been identified in the serum of about 10-15% of patients using standard electrophoresis methods. Initial reports indicate that monoclonal free light chains can be detected in the urine of 60-70% of patients. In B-cell chronic lymphocytic leukaemia monoclonal proteins have been identified by free light chain immunoassay.

Additionally, there are so-called MGUS conditions. These are monoclonal gammopathy of undetermined significance. This term denotes the unexpected presence of a monoclonal intact immunoglobulin in individuals who have no evidence of multiple myeloma, AL amyloidosis, Waldenström's macroglobulinaemia, etc. MGUS may be found in 1% of the population over 50 years, 3% over 70 years and up to 10% over 80 years of age. Most of these are IgG- or IgM-related, although more rarely IgA-related or bi-clonal. Although most people with MGUS die from unrelated diseases, MGUS may transform into malignant monoclonal gammopathies.

In at least some cases for the diseases highlighted above, the diseases present abnormal concentrations of monoclonal immunoglobulins or free light chains. Where a disease produces the abnormal replication of a plasma cell, this often results in the production of more immunoglobulins by that type of cell as that "monoclone" multiplies and appears in the blood.

Immunofixation electrophoresis uses a precipitating antibody against the immunoglobulin molecules. Whilst this improves the sensitivity of the test it cannot be used to quantify monoclonal immunoglobulins because of the presence of the precipitating antibody. Immunofixation electrophoresis is also rather laborious to perform and interpretation may be difficult. Capillary zone electrophoresis is used in many clinical laboratories for serum protein separation and is able to detect most monoclonal immunoglobulins. However, when compared with immunofixation, capillary zone electrophoresis fails to detect monoclonal proteins in 5% of samples. These so-called "false negative" results encompass low-concentration monoclonal proteins.

Total κ and λ assays have been produced. However, total κ and total λ assays are too insensitive for the detection of monoclonal immunoglobulin or free light chain. This is due to high background concentrations of polyclonal bound light chains which interfere with such assays.

More recently, a sensitive assay has been developed that can detect the free κ light chains and separately, the free λ light chains. This method uses a polyclonal antibody directed towards either the free κ or the free λ light chains. The possibility of raising such antibodies was also discussed as one of a number of different possible specificities, in WO 97/17372. This document discloses methods of tolerising an animal to allow it to produce desired antibodies that are more specific than prior art techniques could produce. The free light chain assay uses the antibodies to bind to free λ or free κ light chains. The concentration of the free light chains is determined by nephelometry or turbidimetry. This involves the addition of the test sample to a solution containing the appropriate antibody in a reaction vessel or cuvette. A beam of light is passed through the cuvette and as the antigen-antibody reaction proceeds, the light passing through the cuvette is scattered increasingly as insoluble immune complexes are formed. In nephelometry, the light scatter is monitored by measuring the light intensity at an angle away from the incident light, whilst in turbidimetry light scatter is monitored by measuring the decrease in intensity of the incident beam of light. A series of calibrators of known antigen (i.e. free κ or free I) concentration are assayed initially to produce a calibration curve of measured light scatter versus antigen concentration.

This form of assay has been found to successfully detect free light chain concentrations. Furthermore, the sensitivity of the technique is very high.

The characterisation of the amount or types of free-light chains (FLC), heavy chain or subclasses, or light chain-type bound to heavy chain class or subclass, is important in a wide range of diseases including B cell diseases such as multiple myeloma and other immune mediated diseases such as nephropathy.

WO2015/154052 (Mayo Foundation), incorporated herein in its entirety, discloses methods of detecting immunoglobulin light chains, immunoglobulin heavy chains, or mixtures thereof, using mass spectrometry (MS). Samples comprising immunoglobulin light chains, heavy chains or mixtures thereof are immunopurified, reduced to separate light chains and heavy chains, and subjected to mass spectrometry to obtain a mass spectrum of the sample. This can be used to detect monoclonal proteins in samples from patients. It can also be used to fingerprint, isotype, and identify post-translational modifications such as disulphide bonds and glycosylation in monoclonal antibodies.

WO 2015/131169 (H. Lee Moffitt Cancer Centre) describes methods of monitoring conditions associated with abnormal antibody production. This uses enzymatic cleavage of target immunoglobulin and measuring one or more variable domain peptide fragments by quantitative mass spectrometry. The method is complex because it relies on the identification of variable domain peptide fragments unique to the specific target immunoglobulin associated with the disease, and involves lengthy enzymatic cleavage.

The Applicant of the current invention has realised that free light chain (FLC) specific antibodies and mass spectrometry (MS) can be used in a selective, rapid assay to identify plasma cell associated disease. Unexpectedly they have found that the technique is sensitive enough to detect FLC in normal patients (95% normal reference range for free kappa light chains is 3.3-19.4 mg/L and for free lambda light chains is 5.7-26.3 mg/L). Prior art techniques look at considerably higher levels of immunoglobulins, such as IgG (Adult normal levels are typically 6-16g/L).

Anti-FLC antibodies are used to purify FLC from a sample to reduce contaminants in the assay. The Applicant realised that when the purified FLC from a normal sample is analysed by MS, a curve of different sized and charged FLC is produced. Figure 1 shows the typical result for anti-free lambda, Figure 2 for anti-free kappa and Figures 3 and 4 the overlapping and combined curves of sizes for normal kappa and lambda production. The curves are made up of the many different individual peaks of FLCs produced by normal antibody producing cells in healthy subjects. The Applicant realised that in a monoclonal disease there is an increased amount of a FLC(s) produced due to the multiplying of that clone(s). This raises the amount of FLC considerably above the background normal FLC production. This can be seen as a sharp peak in intensity (see Figure 5). It also increases the sensitivity of the assay compared to prior art systems.

Conventionally the ratio of kappa and lambda is measured to identify abnormal FLC production. This requires the separate measurement and accurate quantifying of kappa and lambda FLC to produce the ratio. This also relies on the amount of one of kappa and lambda being raised relative to the other type of chain, and not having the other raised, for example, where multiple clones are present, distorting the ratio.

The current invention does not require separate kappa and lambda FLCs to be quantified. It instead relies on the single detection of an increased peak compared to the background FLC production. This allows, for example, great sensitivity to be achieved to identify non-secretary multiple myelomas and AL amyloidosis. It also allows the FLCs to be determined without reducing intact immunoglobulins to release light chains bound to heavy chains.

FLCs are present in considerably lower concentrations, for example less than 40 mg/L, such as ca 26mg/L compared to intact immunoglobulins (typically 6 to 16g/L) for adults. 95% normal reference range for free kappa light chains is 3.3-19.4 mg/L and for free lambda light chains is 5.7-26.3 mg/L. It was therefore surprising to be able to identify FLCs even in normal patients and identify the presence of monoclonal FLCs in MGUS patients.

The invention provides a method of identifying or monitoring a plasma cell associated disease comprising purifying immunoglobulin free light chains (FLCs) from a sample from a subject with anti-FLC specific antibodies or fragments thereof and subjecting the purified sample to a mass spectrometry technique to identify the presence of one or more peaks corresponding to one or more monoclonal FLCs in the sample.

That is the mass spectrometry assay typically separates the free light chains by virtue of their charge and mass, when subjected to mass spectrometry. This typically produces a normal distribution of FLCs with different molecular masses reflecting the molecular masses of the germline light chain amino acid sequences and the somatic hypermutation of those sequences in subjects with normal FLC. As discussed above, the presence of a monoclonal FLC produces a peak resulting from the increased amount of the monoclonal FLC produced by the plasma cell associated disease. That monoclonal FLC has a size and charge and is identified by the increased amount (peak) compared to the background normal FLCs present.

Mass spectrometry (MS), as used herein includes, for example, liquid chromatography - mass spectrometry (LC-MS), microflow liquid chromatography electrospray ionisation coupled to a quadruple time-of-flight mass spectrometry (micro LC-ESI-Q-TOF MS). This may include, for example, the use of positive ion mode. An Orbitrap mass spectrometer, ion trap mass spectrometer, time-of-flight mass spectrometer, triple quadrupole mass spectrometer, or quadrupole mass spectrometer may be used.

Alternatively, the MS technique includes a matrix assisted laser desorption ionisation-time-of-flight mass spectrometry (MALDI-TOF-MS). When MALDI-TOF is used, it is typically used on positive mode for charged ions, preferably 1+, 2+ and/or 3+ ions, and most preferably for 2+ ions.

Typically no enzymatic cleavage of the FLC is carried out.

The anti-FLC specific antibodies or fragments thereof may be monoclonal or polyclonal antibodies or fragments. The antibodies may be synthetic antibodies, synthetic antibodies include recombinant antibodies, nucleic acid aptamers and non-immunoglobulin protein scaffolds.

The antibodies may be species specific, such as anti-human or anti-horse or anti-sheep or anti-pig. The antibody may be raised in cartilaginous fish, sheep, goat, horse, rabbit, cow, camelids such as llamas, rats or mouse. The antibodies or fragments are capable of specifically binding to free light chains.

The fragment of the antibody may, for example, be F(ab')₂fragment.

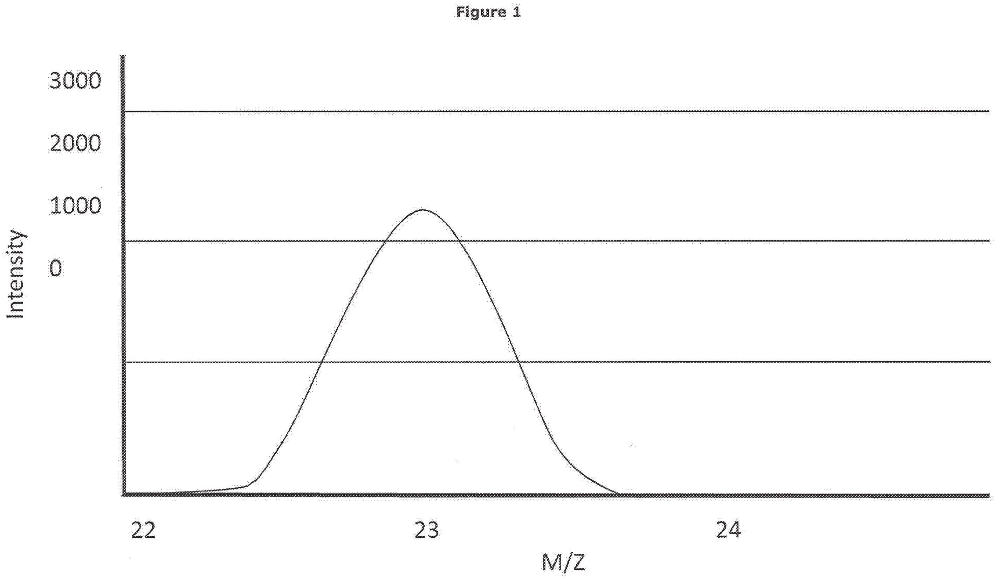

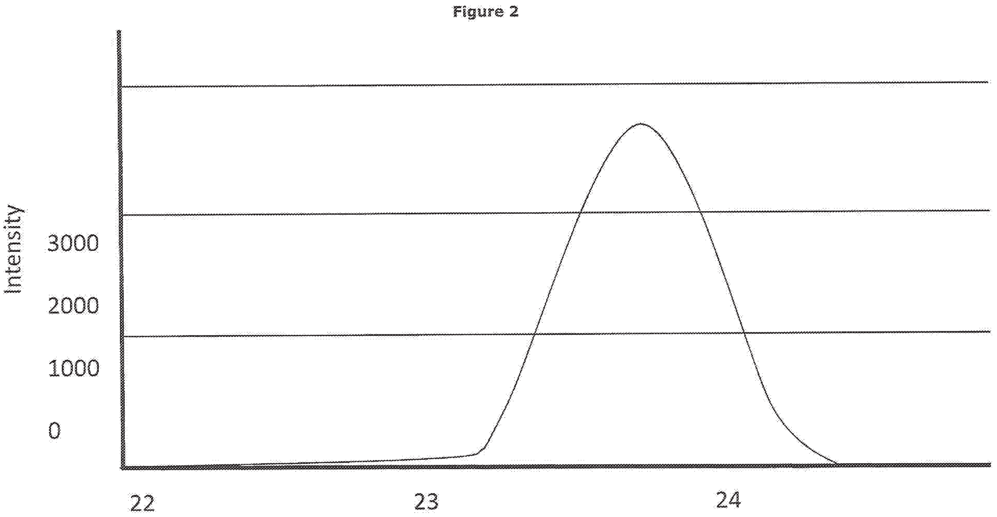

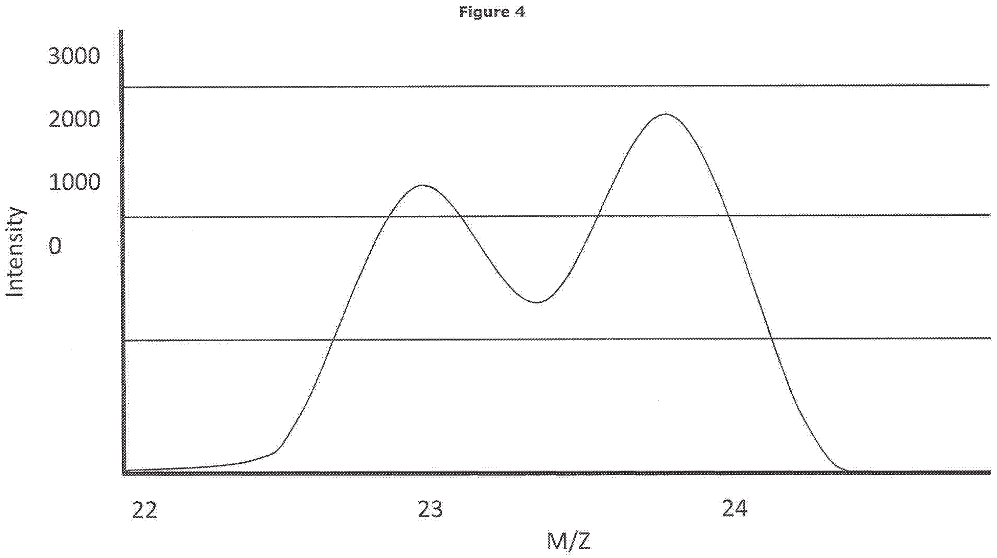

The anti-FLC specific antibodies or fragments may be anti-kappa FLC specific or anti-lambda FLC specific. That is, the lambda FLCs and kappa FLCs may be separated separately from the sample. For example, two separate assays would then be run on the mass spectrometry. Separate lambda FLC recording and a separate kappa FLC recording, such as those shown in Figures 1 and 2, would then be produced
Alternatively, or more typically, a mixture of anti-kappa FLC specific and anti-lambda specific antibodies or fragments are used. This copurifies both the lambda and kappa free light chains, to produce, for example, the readout shown in Figure 4 for normal healthy patients. The monoclonal peaks are still identified above the combined background FLCs.

The anti-FLC specific antibodies or fragments thereof may comprise one or more non-disulphide cross links between at least one heavy chain (or fragment) and at least one light chain (or fragment) of the antibody or fragments thereof.

The cross-link typically comprises a thioether bond. Alternative cross-links may also be used.

A thioether cross-link comprises a thioether bond. This is a link between residues of the antibody wherein the link has a single sulphur bond rather than a disulphate bond. That is thioether cross-links do not include links that comprise more than one sulphur atom, such as disulphide bridges that are familiar to those skilled in the art. Instead, a thioether cross-link comprises a single sulphur bond that bridges residues of a macromolecule. One or more additional non-sulphur atoms may additionally form the link.

The residues linked by thioether cross-links can be natural residues or non-natural residues. Formation of the thioether cross-link can result in a loss of atoms from the residues, as will be recognised by those skilled in the art. For example, formation of a thioether cross-link between side chains of two cysteine residues can result in loss of a sulphur atom and hydrogen atoms from the residues, yet the resulting thioether cross-link will be recognised as linking the cysteine residues by one skilled in the art.

Thioether cross-links can link any two residues of the antibody. One or more of the residues may be selected, for example, from cysteine, aspartic acid, glutamic acid, histidine methionine and tyrosine. Two of the residues may be selected from the group consisting of cysteine, aspartic acid, glutamic acid, histidine, methionine and tyrosine. More typically two of the residues are cysteine residues. Typically, only one thioether cross-link is between the heavy chain and the light chain. Alternatively, two, three or more thioether cross-links may be used. The heavy chain pair of the antibody, or a fragment thereof, may also be linked by one or more non-disulphide cross-links, such as thioether bonds.

Thioether cross-links are described in, for example, WO2006/099481, and Zhang et al (2013) J. Biol. Chem. vol 288(23), 16371-8 and Zhang & Flynn (2013) J. Biol. Chem, vol 288(43), 34325-35 incorporated herein by reference.

Phosphines and phosphites may be used. Here, 'Phosphine' refers to any compound containing at least one functional unit with the general formulae R₃P (where P = phosphorous and R = any other atom). In phosphites, the R positions are occupied specifically by oxygen atoms. R₃P-containing compounds act as strong nucleophiles that can attack disulphide bonds. This can result in reduction of disulphides, however under some conditions, may also result in thioether bond formation.

Compounds include:
Tris(dimethylamino)phosphine (CAS Number 1608-26-0)
Tris(diethylamino)phosphine (CAS Number 2283-11-6)
Trimethylphosphite (CAS Number 121-45-9)
Tributylphosphine (CAS Number 998-40-3)

### References: Bernardes et al. (2008) Angew. Chem. Int. Ed., vol 47, 2244-2247 incorporated herein by reference

Cross-links may also comprise cross-linkers such as a maleimide cross-linker, which reacts with free thiols to cross-link to chains of the antibody molecule. This can be made to bind on one side of a thiol group and additionally on another moiety such as a lysine carboxyl group, as described in WO00/44788.

Bi-functional cross-linkers may be used comprising two reactive moieties linked together by a linker, especially a flexible linker. The linker may comprise one or more carbons covalently bound together in a chain, for example a substituted or non-substituted alkyl. The linker especially a C1-C10, most typically a C2-C6 or C3-C6 linker. The Applicants have found that C2-C6 containing cross linkers, such as, α,α'-Dibromo-*m*-xylene, BMOE (bismaleimidoethane) or BMB (bismaleimidobutane) particularly useful with relatively high levels of recovery of cross-linked protein.

### Bismaleimide is a homobifunctional sulfhydryl reactive crosslinker

This is a well characterised class of cross-linker contains two maleimide groups connected by a hydrocarbon or other linker. The maleimide groups spontaneously react with free sulfhydryl groups exposed by reduction of disulphides to form a non-reducible thioether bond at each sulfhydryl, thereby covalently crosslinking the two remaining cystines.

Compounds include:
Bis(maleimido)ethane (CAS Number 5132-30-9)
1,4-bis(maleimido)butane (CAS Number 28537-70-4)

### References:

Auclair et al. (2010) Strategies for stabilizing superoxide dismutase (SOD1), the protein destabilized in the most common form of familial amyotrophic lateral sclerosis. Proc Natl Acad Sci U S A, vol 107(50) - pages 21394-9
Geula at al. (2012) Structure-based analysis of VDAC1 protein: defining oligomer contact sites. J Biol Chem, vol 287(3), 475-85
Kida et al. (2007) Two translocating hydrophilic segments of a nascent chain span the ER membrane during multispanning protein topogenesis. J Cell Biol, vol 171(7) pages 1441-1452 incorporated herein by reference
*α,α'-Dibromo-m-xylene is a homobifunctional sulfhydryl reactive crosslinker which may also be used*
Dibromo-m-xylene (CAS Number 626-15-3) is a member of the di-alkyl halide class of compounds and acts as a homobifunctional crosslinker that reacts with free sulfhydryl groups.

### Reference:

Jo et al. (2012) Development of α-Helical Calpain Probes by Mimicking a Natural Protein-Protein Interaction J Am Chem Soc., col 134(42) - pages 17704-13 incorporated herein by reference.

Alternative sulfhydryl reactive cross-linking compounds forming stable thioether bonds

There are at least six classes of reagent known to react with free sulfhydryls and result in a non-reducible covalently cross-linked product. The specific reactivity of these compounds to sulfhydryl groups varies and some will react with water, amines and carboxyl groups under certain conditions. In addition, many of these compounds have bulky linker groups, which may limit their ability to cross-link in restricted spatial environments. The list below gives a few examples from each class, but a more comprehensive list and references is given in:
Chemistry of Protein Conjugation and Cross-linking, Wong, S: ISBN 0-8493-5886-8 incorporated herein by reference
Bismaleimides
   bis(maleimido)hexane; N-N'-Methylenebismaleimide; Bis(N-maleimidomethyl)ether; N,N'-(1,3-Phenylene)-bismaleimide; Bis(N-maleimido)-4,4'-bibenzyl; Naphthalene-1,5-dimaleimide
Haloacetyl derivatives
   1,3-Dibromoacetone; N,N'-Bis(iodoacetyl)polmethylenediamine; N,N'-Di(bromoacetyl)phenylhydrazine; 1,2-Di(bromoacetyl)amino-3-pheylhydrazine; γ-(2,4-Dinitrophenyl)-α-bromoacetyl-L-diaminobutyric acid bromoacetyl-hydrazide
Di-alkyl halides
   α ,α'-Dibromo-*p*-xylene sulfonic acid; α,α'-Diiodo-*p*-xylene sulfonic acid; Di(2-chloroethyl)sulphide; Tri(2-chloroethyl)amine; N,N-Bis(β-bromoethyl)benzylamine
2.4 s-Triazines
   Dichloro-6-methoxy-s-triazine; 2,4,6-Trichloro-s-triazine (Cyanuric acid); 2,4-Dichloro-6-(3'-methyl-4-aminoanilino)-*s*-triazine; 2,4-Dichloro-6-amino-s-triazine
Aziridines
   2,4,6-Tri(ethyleneimido)-s-triazine; N,N'-Ethyleneiminoyl-1,6-diaminohexane; Tri[1-(2-methylaziridenyl)]-phosphine oxide
Bis-epoxides
   1,2:3,4-Diepoxybutane; 1,2:5,6-Diepoxyhexane; Bis(2,-epoxypropyl)ether; 1,4-Butadioldiglycidoxyether

The cross-link may replace one or more naturally occurring disulphide bonds or alternatively may be produced in addition to the disulphide bond.

Other cross linking chemistries are also possible including . Alternative cross linking may include the following:

### Carboxyl to Primary Amine

(a) Carbodiimide activation: N-(3-Dimethylaminopropyl)-N'-ethylcarbodiimide HCl (EDC; CAS Nr 25952-53-8.
(b) Carbodiimide activation: EDC stabilised with N-Hydroxysulfosuccinimide (sNHS; CAS Nr 106627-54-7)
(c) 4-(4,6-dimethoxy-1,3,5-triazin-2-yl)-4-methylmorpholinium (DMTMM; CAS Nr 3945-69-5)
   - Once activated with any of the above, Carboxylates will react with primary amines (Lysine, N-terminus) to form a covalent amide bond
   - Note: under some conditions, EDC/sNHS activation can lead to covalent ester bond formation between activated carboxyl groups (on Aspartic acid, Glutamic acid, C-terminus) and hydroxyl groups (i.e. Serine, Threonine and Tyrosine)

### Carboxyl to Carboxyl

(a) Activate carboxyl with EDC, EDC/sNHS or DMTMM and then crosslink with Amine-derivatized Polyethylene Glycol e.g. Amine-PEGn-Amine, where n = number of repeating PEG units)
(b) Activate carboxyl with DMTMM and crosslink with homobifunctional hydrazide e.g. Adipic acid dihydrazide (ADH; CAS Nr 1071-93-8)

### Carboxyl to Sulfhydryl

If disulphides present, reduce to -SH with reducing agent, e.g. Tris(2-carboxyethyl)phosphine hydrochloride (TCEP; CAS Nr 51805-45-9). Activate carboxyl with Carbodiimide (EDC) and crosslink with:
(a) 3-(4-(4-(Aminomethyl)-1H-1,2,3-triazol-1-yl)phenyl)propiolonitrile hydrochloride (APN; CAS Nr 1643841-88-6)
(b) Amine-(PEG)n-Maleimide, where n = number of repeating PEG units (MAL-PEG-NH2)

### Amine to Amine

(a) PEGylated bis(sulfosuccinimidyl)suberate) e.g. BS(PEG)5, where 5 = number of repeating PEG units
(b) Dimethyl Pimelimidate (DMP; CAS Nr 58537-94-3)
(c) *p*-Phenylene diisothiocyanate (PDITC; CAS Nr 4044-65-9)
(d) Suberic acid bis(N-hydroxysuccinimide ester) (DSS; CAS Nr 68528-80-3)
(e) Ethylene glycol bis(sulfosuccinimidyl succinate) (Sulfo-EGS; CAS Nr 167410-92-6)

### Amine to Sulfhydryl

(a) Maleimide-PEG8-succinimidyl ester (CAS Nr 756525-93-6)
(b) 4-(N-Maleimidomethyl)cyclohexanecarboxylic acid N-hydroxysuccinimide ester (SMCC; CAS Nr 64987-85-5)
(c) 3-(2-Pyridyldithio)propionic acid N-hydroxysuccinimide ester (SPDP; CAS Nr 68181-17-9)
(d) 3-(4-formylphenyl)propiolonitrile (APN-CHO)
(e) Iodoacetic acid N-hydroxysuccinimide ester (IAA-NHS; CAS Nr 39028-27-8)

### Hydroxyl to Sulhydryl

(a) 4-(Maleinimido)phenyl isocyanate (PMPI; CAS Nr 123457-83-0)

### Other Chemistries

(a) *p*-Azidophenylglyoxal (APG; CAS Nr 1196151-49-1)
   • Reacts with Arginine and to lesser extent with Cystine (Disulphide bonds) and Histidine. Upon photoactivation, initiates addition reactions with double bonds, C-H and N-H or with primary amines via ring expansion mechanism.
(b) 1,4-Butanediol diglycidyl ether (BDDE; CAS Nr 2425-79-8)
   • Reacts with hydroxyls, amines and sulfhydryl groups
(c) 4-(4-diazoniophenyl)benzenediazonium (CAS Nr 5957-03-9
   • Reacts with tyrosine and histidine
(d) Benzophenone-4-iodoacetamide (CAS Nr 76809-63-7)
   • Reacts with sulfhydryls and upon photoactivation will react with active C-H and N-H bonds to form covalent bonds
(e) Succinimidyl 2-[(4,4'-azipentanamido)ethyl]-1,3'-dithiopropionate) (SDAD; CAS Nr 1253202-38-8
   • NHS ester group reacts with primary amines; diazirine group reacts efficiently with any amino acid side chain or peptide backbone upon photoactivation

Typically at least 50%, at least 60 %, at least 70 %, at least 80%, at least 90 % or at least 95% of the antibodies are cross-linked. Cross linking efficiencies of 70% - 80% have been observed using, for example, bismaleimide. The cross-linked antibodies may be further purified to produce higher levels of cross-linking, for example by adding a reducing agent to break the disulphide bonds of remaining non-cross-linked antibodies and separating using, for example, gel electrophoresis.

The advantage of using cross-linked antibodies is that it reduces contamination of the sample by, for example, free light chains that have been released from the purifying antibodies. This increases the sensitivity and accuracy of the system.

The method may additionally comprise the step of purifying total kappa and total lambda light chains with anti-total kappa and anti-total lambda antibodies or fragments thereof, and subjecting the purified sample to mass spectrometry to identify the presence of one or more peaks corresponding to monoclonal light chain production. That is, whilst the anti-FLC specific antibodies substantially only bind free light chains, the total kappa and/or total lambda light chains bind to both free light chains and also light chains which are bound to heavy chains. This therefore detects any light chains in the sample, not just free light chains. This additional step assists in identifying, for example, MGUS where monoclonal intact immunoglobulins are produced.

Again, separate total kappa light chain-specific antibodies and total lambda light chain-specific antibodies may be used, or alternatively a mixture of the two antibodies together may be used. The antibodies and fragments may be as defined above, or modified as above. Typically they are cross-linked with one or more non-disulphide cross-links.

An alternative method which may be used instead of or in addition to assaying using total kappa and/or lambda antibodies is subject to a portion of the sample to reducing conditions, for example using a reducing agent. This releases the bound light chains from heavy chains. The released light chains may then be enriched or purified using anti-total kappa and/or lambda light chains or using anti-kappa and/or anti-lambda FLC antibodies.

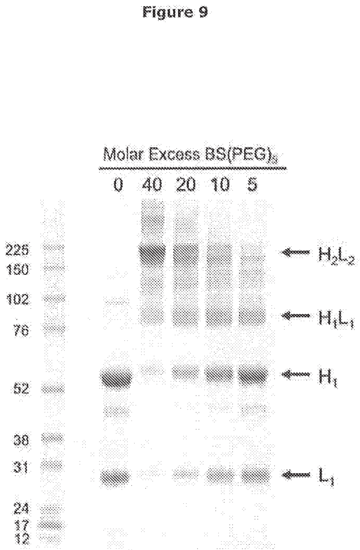

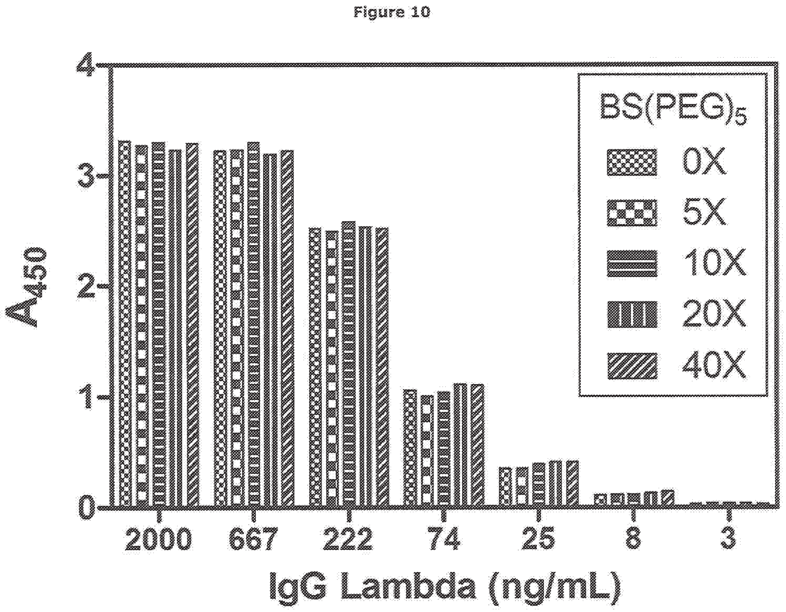

If the antibodies used in the enriching step are modified by the presence of one or more non-disulphide cross-links between the light chain and heavy chain of the detecting antibodies then they may still be used under reducing conditions to detect the released light chains in the sample as shown in Figures 9 and 10.

The sample may be a suitable bodily fluid including, for example, tear fluid, plasma, serum, blood, urine, saliva or cerebrospinal fluid.

The plasma cell associated disease may be any one in which the disease produces one or more monoclonal light chains. These include, for example: intact immunoglobulin, multiple myeloma, light chain multiple myeloma, non-secretory multiple myeloma, AL amyloidosis, light chain deposition disease (LCDD), smouldering multiple myeloma, monoclonal gammopathy of undetermined significance (MGUS), macroglobulinemia, POEMS (polyneuropathy, organomegaly, endocrinopathy, monoclonal gammopathy and skin changes) syndrome.

The antibodies or fragments used in the invention to purify the FLC or total light chains may, for example, be provided on a suitable immunopurification column of the types generally known in the art. Alternatively, the antibodies may be attached to magnetic beads, for example of the type known as "DynaBeads^{™}". This allows the antibodies to be mixed with the sample to bind to the FLC or total light chains in the sample. The antibodies attached to the FLC or light chains are then removed from the sample with the aid of a magnet to which the antibodies or fragments are attracted.

The FLC or light chains may then be eluted from the antibody and used within the mass spectrometer, for example, by placing on a mass spectrometry target.

Alternatively, the antibodies or fragments may be immobilised on, for example, a mass spectrometry target. The sample is contacted with the target comprising the antibodies, the target is washed to remove unbound material, and then the mass spectrometer target, containing the bound FLC or light chain (via the antibodies of fragments) is then subjected to mass spectrometry to detect the presence of the bound free light chain or light chains.

Accordingly, a further aspect of the invention provides anti-kappa FLC antibodies or fragments thereof, anti-lambda FLC antibodies or fragments thereof, and at least one mass spectrometry target. Typically the antibodies are immobilised on the mass spectrometry target. Typically a mixture of anti-kappa FLC and anti-lambda FLC antibodies are immobilised on the target.

Mass spectrometers comprising a mass spectrometry target as defined above are also provided.

The methods of the invention may also be used in combination with one or more additional assays to further characterise any conditions that the subject has. For example:
Serum plasma electrophoresis or immunofixation electrophoresis may be carried out to further characterise the condition.

Total protein albumin or beta-2-microglobulin may be detected by MS or conventional assays known in the art.

Renal function markers such as creatinine and cystatin may be assayed. Cardiac markers may also be assayed such as troponin, NT-pro-BNP. Bone profile/turnover for hypercalceamia may be assayed as might alkaline phosphatase (ALP) and phosphate (Ph).

Accordingly, the presently claimed invention is expected to detect a variety of different plasma cell associated diseases including intact immunoglobulin, multiple myeloma, light chain multiple myeloma, non-secretory multiple myeloma, AL amyloidosis, light chain deposition disease, smouldering multiple myeloma, plasmacytoma and MGUS. MGUS will be detected when a normal FLC clone is present.

The following table shows the sensitivity of the most relevant symptomatic monoclonal gammopathy screening panels, compared to the expected sensitivity of the current invention. SPE is serum plasma electrophoresis, sFLC is serum free light chains, MS FLC is the current invention.

| **Diagnosis** | **SPE (%)** | **sFLC (%)** | **SPE & sFLC (%)** | **MS FLC (%)** |
|---|---|---|---|---|
| **All** | 79.0 | 74.3 | 94.3 | >99% |
| **MM** | 87.6 | 96.8 | 100.0 | >99% |
| **Macroglobulinemia** | 100.00 | 73.1 | 100.0 | >99% |
| **ASMM** | 94.2 | 81.2 | 99.5 | >99% |
| **Plasmacytoma** | 72.4 | 55.2 | 86.2 | >99% |
| **POEMS** | 74.2 | 9.7 | 74.2 | >99% |
| **Primary AL** | 65.9 | 88.3 | 96.2 | >99% |
| **LCDD** | 55.6 | 77.8 | 77.8 | >99% |

The invention will now be described by way of example only, with reference to the following figures:
**Figure 1** shows a mass spectrometry run in positive ion mode covering singly charged ion range 22.5 to 23.5 kDa of normal samples without the presence of a plasma cell associated disease, following purification with anti-free lambda antibodies.
**Figure 2** shows a mass spectrometry run in positive ion mode covering the singly charged ion range 22.5 to 23.5 kDa of normal samples following purification with anti-free kappa antibodies.

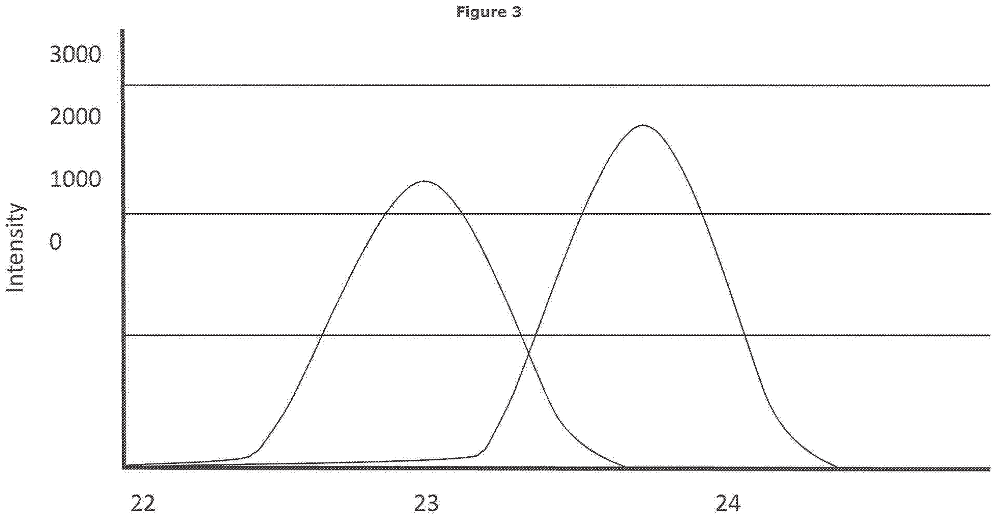

**Figure 3** shows an overlay of the printouts for Figures 1 and 2.
**Figure 4** shows the effect of co-purification with anti-free lambda and anti-free kappa antibodies on a normal sample.

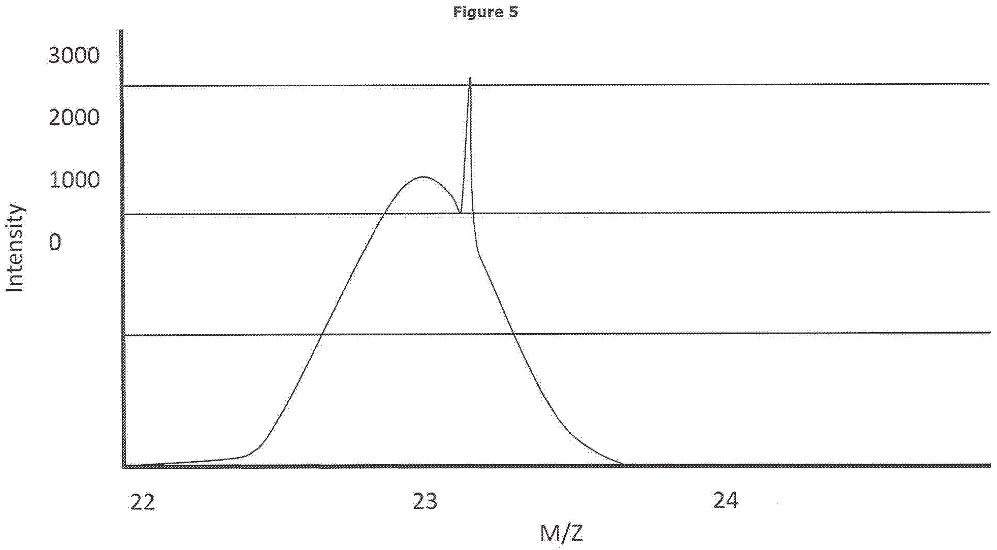

**Figure 5** shows a mass spectrometry run with an abnormal sample following purification with anti-free lambda. This shows an abnormal peak showing the presence of the abnormal monoclonal protein.

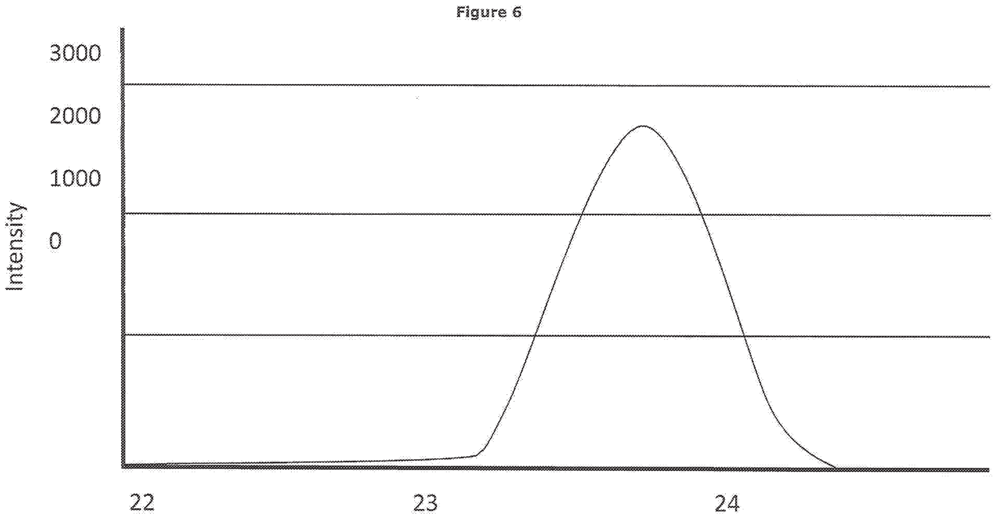

**Figure 6** shows a mass spectrometry run of an abnormal sample where the abnormal clonal production of free lambda is present, following purification with anti-free kappa.

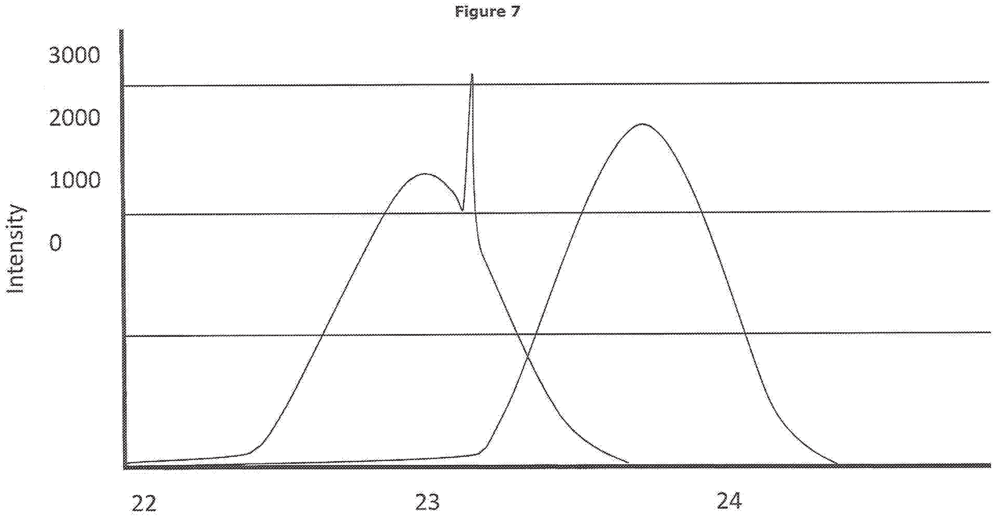

**Figure 7** shows the overlay of the printouts shown in Figures 5 and 6.

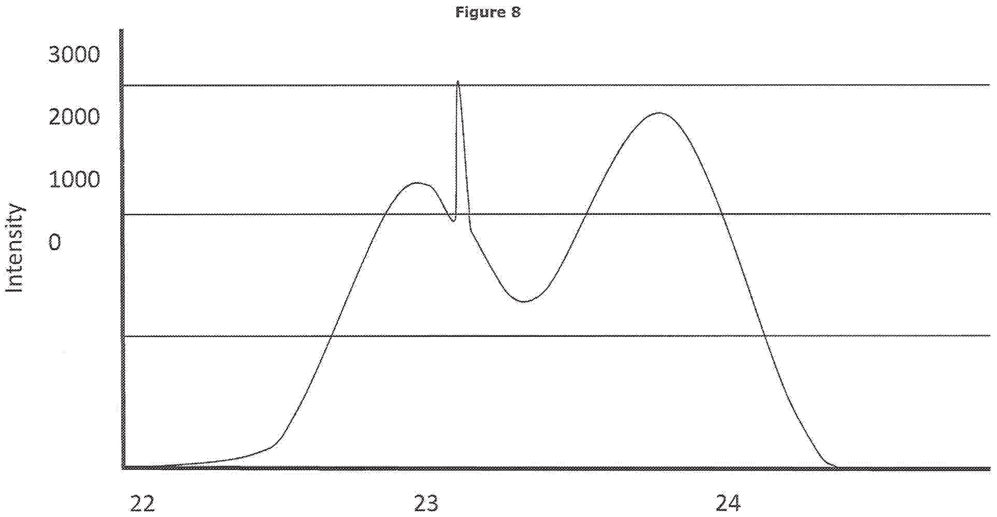

**Figure 8** shows a mass spectrometry run of an abnormal sample comprising abnormal clonal production of free lambda, following co-purification with anti-free lambda and anti-free kappa.
**Figure 9** Crosslinking of sheep anti-human IgG antibodies by BS(PEG)₅, as shown by reducing SDS-PAGE analysis. L₁ = free immunoglobulin light chain, H₁ = free immunoglobulin heavy chain, H₁L₁ and H₂L₂ = crosslinked heavy and light chain moieties.
**Figure 10** Antibodies crosslinked with BS(PEG)s retain biological activity. Sheep anti-human IgG antibodies were crosslinked with increasing concentrations of BS(PEG)s and analysed for their IgG binding activity by ELISA.

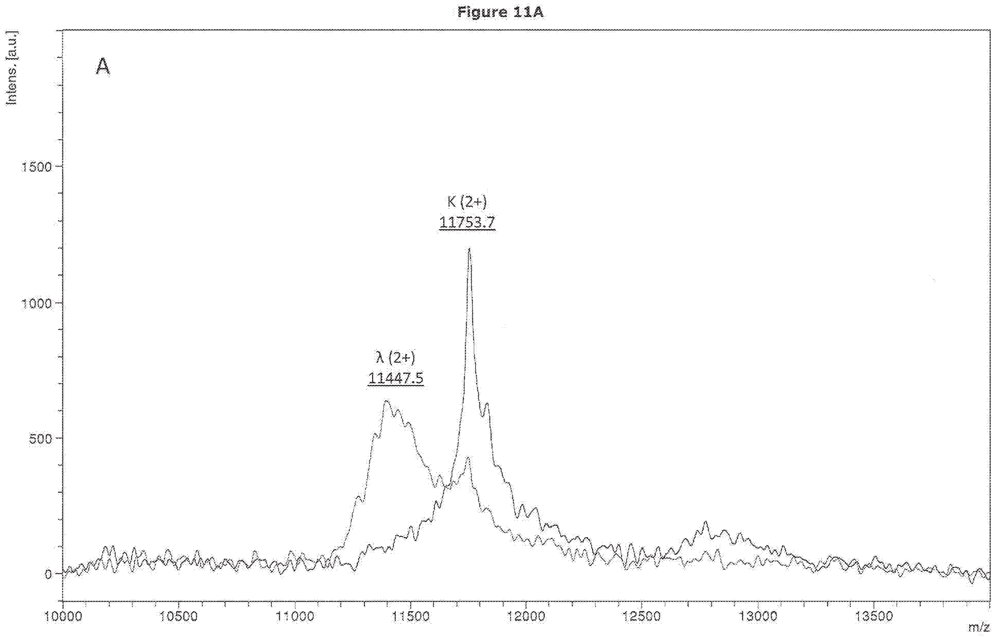

**Figure 11A** shows a mass spectrometry run for a sample from a subject with IgG kappa MGUS in positive ion mode for double charged ions.

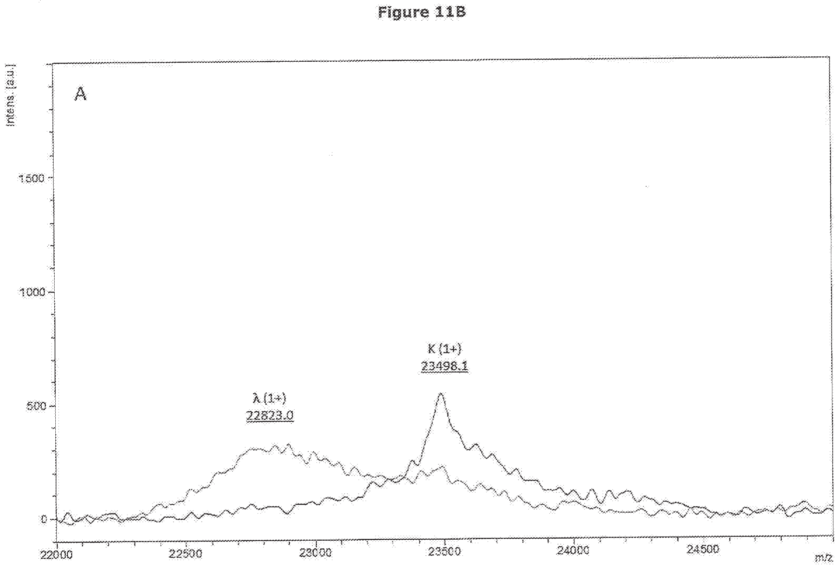

**Figure 11B** shows a mass spectrometry run for a sample from a subject with IgG kappa MGUS in positive ion mode for single charged ions.

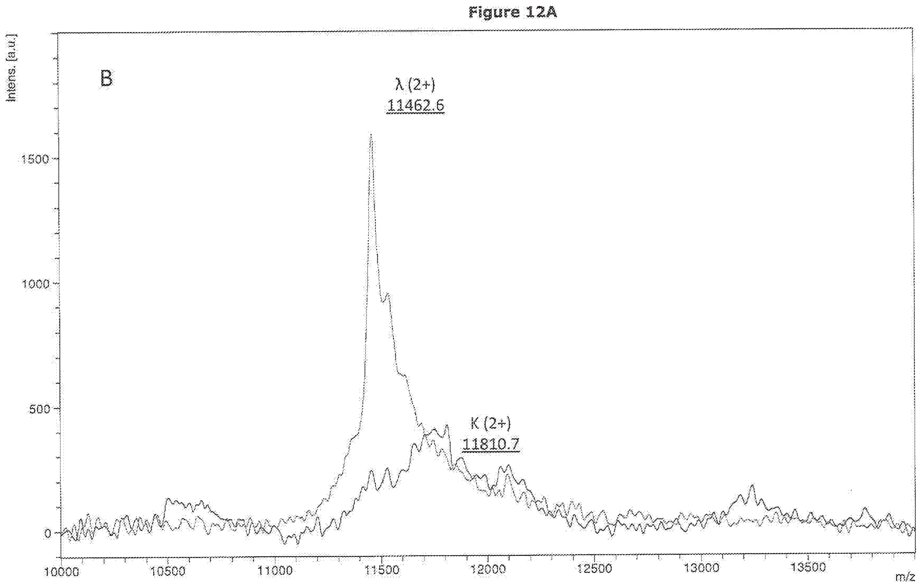

**Figure 12A** shows a mass spectrometry run for a sample from a subject with IgA lambda MGUS in positive ion mode for double charged ions.

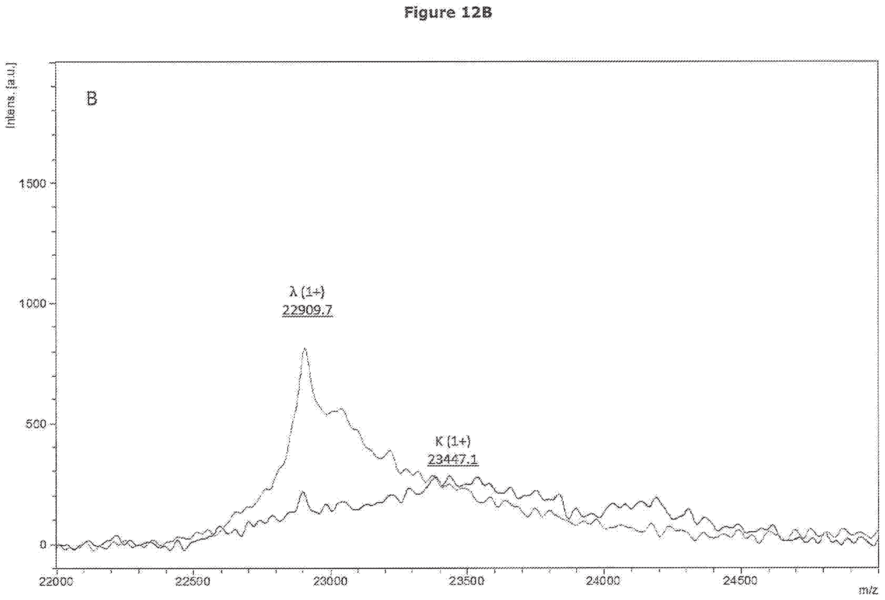

**Figure 12B** shows a mass spectrometry run for a sample from a subject with IgA lambda MGUS in positive ion mode for single charged ions.

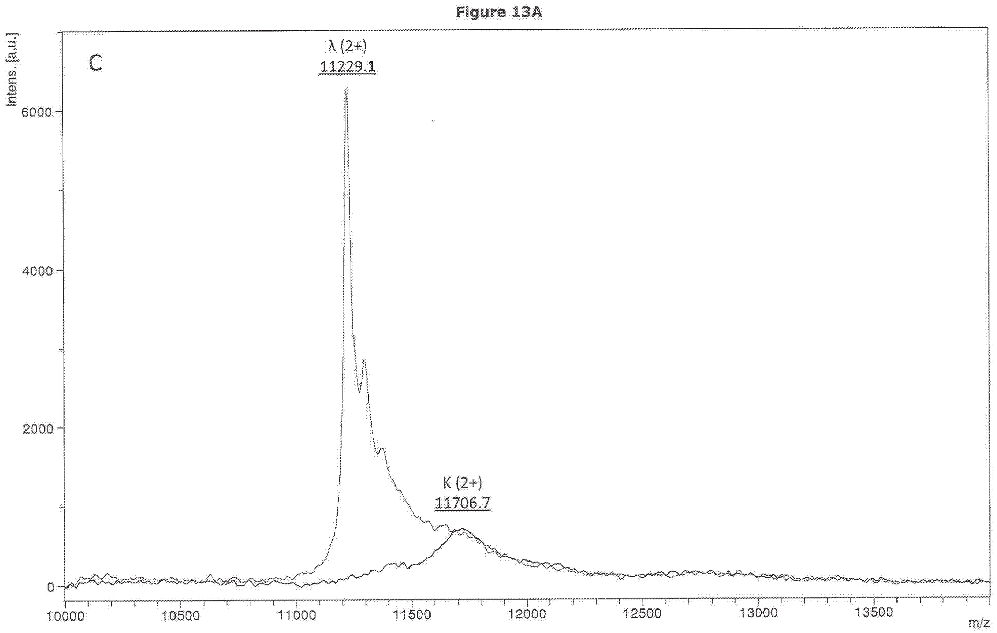

**Figure 13A** shows a mass spectrometry run for a sample from a subject with IgA lambda MGUS in positive ion mode for a double charged ion.

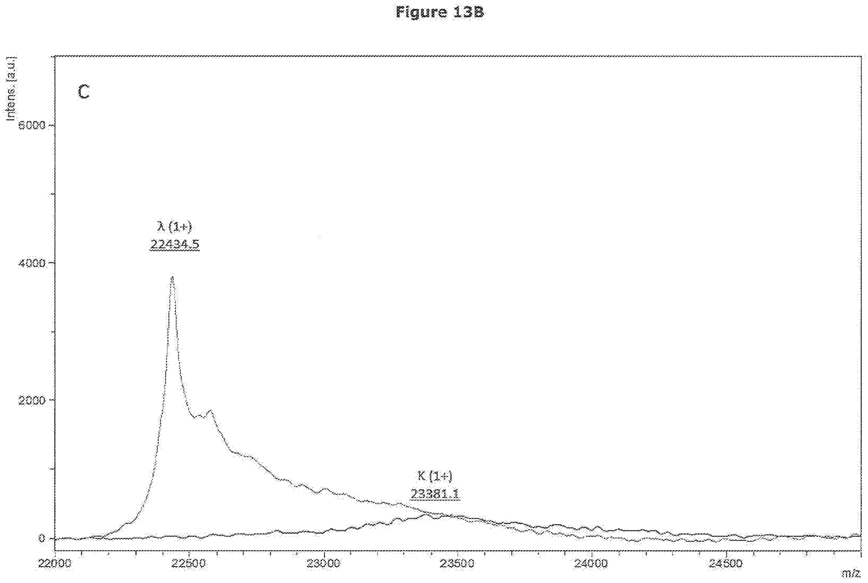

**Figure 13B** shows a mass spectrometry run for a sample from a subject with IgA lambda MGUS in positive ion mode for a single charged ion.

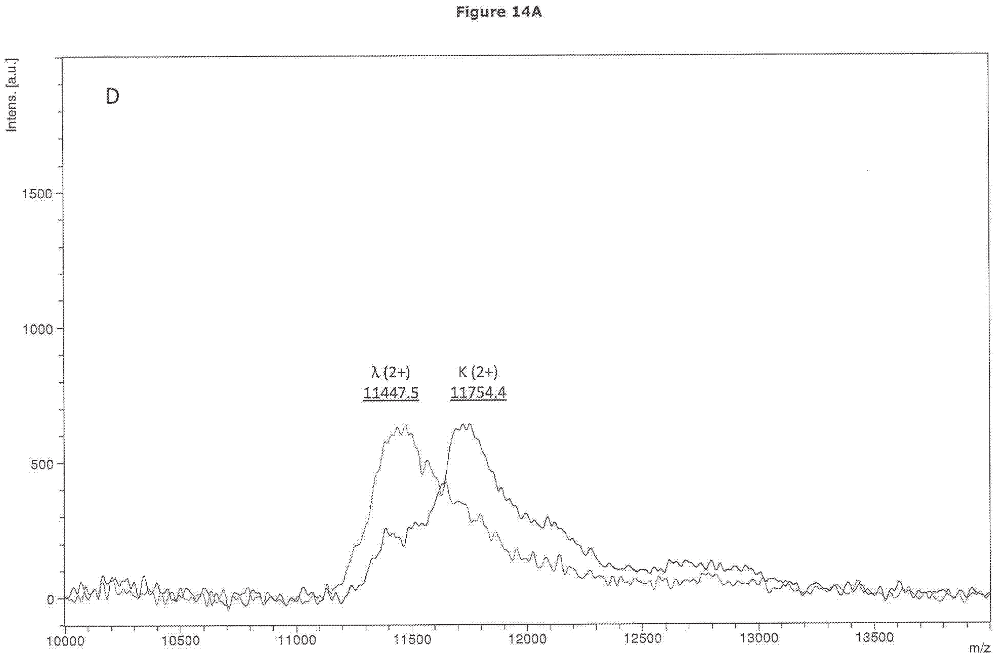

**Figure 14A** shows a mass spectrometry run from a normal subject in positive ion mode for double charged ions.

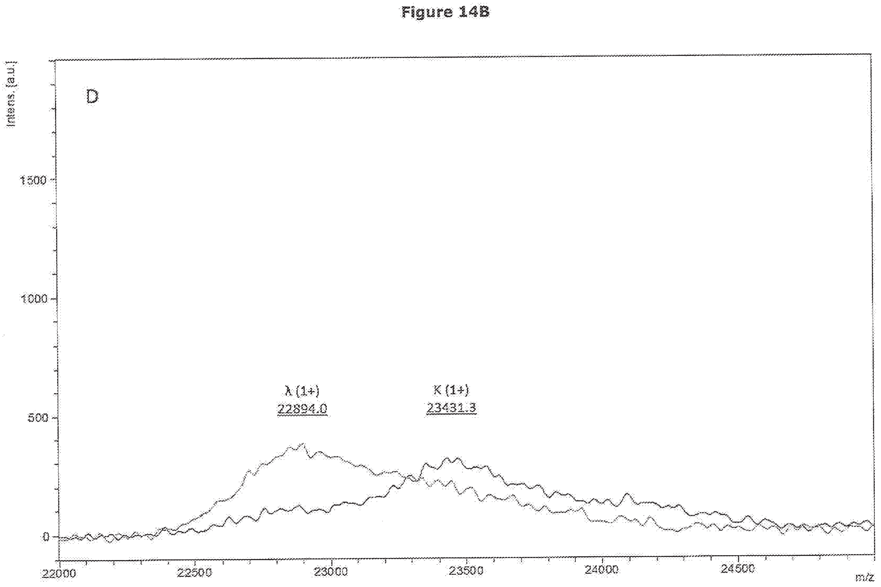

**Figure 14B** shows a mass spectrometry run of the normal sample in Figure 14A in positive ion mode for single charged ions.

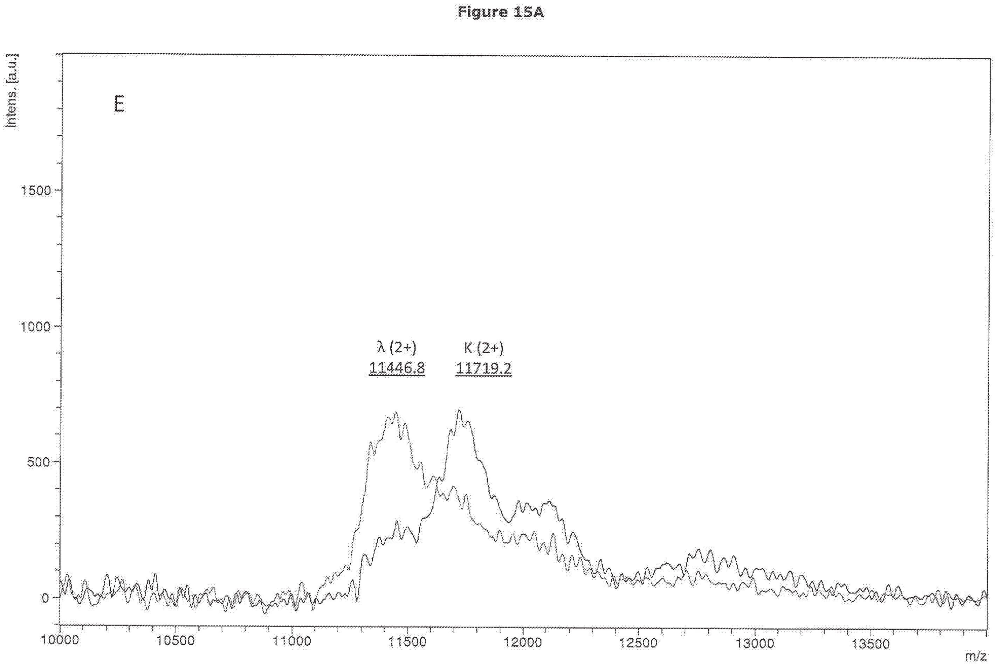

**Figure 15A** shows a mass spectrometry run of a normal sample in positive ion mode for double charged ions.

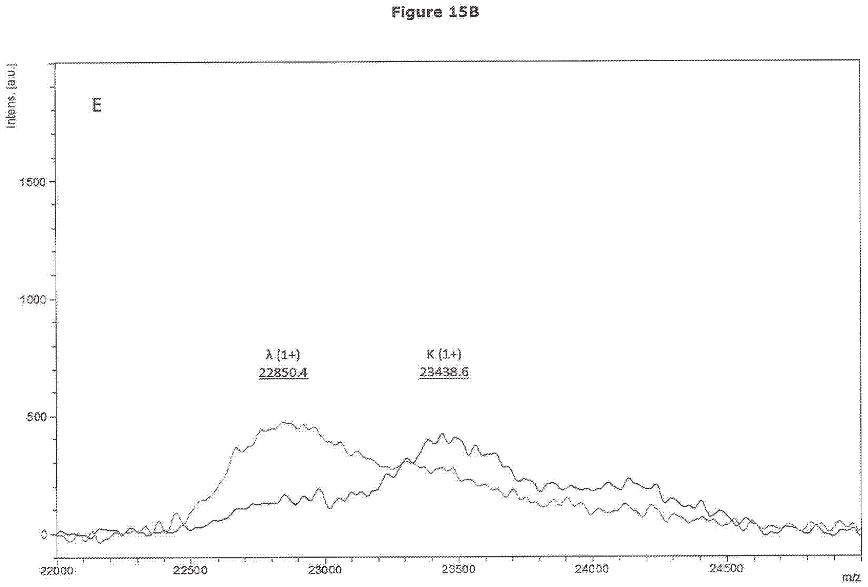

**Figure 15B** shows a mass spectrometry run of the normal sample in Figure 15A in positive ion mode for single charged ions.

Kappa FLC and lambda FLC can be purified either separately or co-purified, using anti-kappa FLC antibodies and anti-lambda FLC antibodies, or mixtures thereof.

The purified FLCs are spotted onto a mass spectrometry plate and analysed by MALDI-TOF.

Figures 1 to 8 show how the presence of a monoclonal free light chain in the serum of a patient, may be easily identified by the presence of a peak, above the background, normal, production of free light chains. This peak may be identified even in areas of where there is overlap between kappa and lambda peaks.

Anti-human IgG antibodies can be crosslinked by the homobifunctional crosslinker BS(PEG)₅.

The antibodies were incubated with increasing concentrations of BS(PEG)s (0 -40 molar excess) and analysed by reducing SDS-PAGE analysis. As shown in Figure 9, in the absence of BS(PEG)₅, the reducing agent (50 mM DTT) leads to the disassociation of the antibody into its heavy and light chain parts. In contrast, incubation of the antibody with increasing concentrations (0 - 40 Molar excess) of BS(PEG)s produces a concomitant increase in crosslinking of the heavy and light chains to form reduction-resistant heavy-light chain pairs.

Antibodies crosslinked with BS(PEG)s retain biological activity.

Purified human IgG Lambda was coated onto microtitre plates at 3 - 2000 ng/mL. Following crosslinking with 0 - 40 Molar excess of BS(PEG)₅, sheep Anti-human IgG antibodies were applied. The amount of bound antibody was determined using donkey anti-sheep antibodies conjugated to horse radish peroxidase reporter enzyme and 3,3',5,5'-Tetramethylbenzidine chromogenic substrate. As shown in Figure 10, at concentrations of BS(PEG)s up to 40X molar excess, no significant effect on human IgG binding was observed, as compared to the uncrosslinked antibody.

### Testing of Samples from Subjects with MGUS and Normal Samples

Human serum samples (3 IPE positive MGUS Figures 11 to 13 (A-C) and 2 healthy controls) Figures 14 to 15 were diluted with PBS-T buffer (25 mM Sodium phosphate, 150 mM NaCl, 0.1% tween 20, pH 7.0) and incubated with antibody coated magnetic beads resuspended and washed sequentially 3 x in PBS-T and twice with deionised water. The beads were eluted with an acidic buffer for 15 mins at RT. One of the elution was mixed with a matrix (a-cyano-4-hydroxycinnamic acid, 10 mg/gl) and then spotted onto a polished steel MALDI target plate using the Mosquito HTS spotter (TTP), and analysed on the Bruker Biotyper MALDI-TOF mass spectrometer (Microflex LT/SH Smart). Mass spectra were acquired in positive ion mode covering the m/z range of 10,000 to 30,000 which includes the singly charged (+1, m/z 22-25 kDa) and doubly charged (=2, m/z 10-14 kDa) ions. Data was analysed with Bruker Flex analysis software.

| Figure | Sample Description | IFE | Kappa (mg/L) | Lambda (mg/L) | FLC Ratio |
|---|---|---|---|---|---|
| 11 | MGUS with abnormal FLC ratio | IgG kappa | 57.73 | 6.95 | 8.31 |
| 13 | MGUS with abnormal FLC ratio | IgA lambda | 12.82 | 153.20 | 0.08 |
| 12 | MGUS with normal FLC ratio | IgA lambda | 5.36 | 19.43 | 0.28 |

This shows that using mass spectrometry it is possible to detect monoclonal kappa and lambda free light chains even at the free light chain levels normally seen in normal samples.

The preliminary results also show that using the positive mode for double charged ions allows the abnormal monoclonal FLCs to be detected better than the single positive mode.

The ability to rapidly identify the presence of low levels of FLCs in samples, even in subject with MGUS which are often difficult to identify, is surprising and opens up a new approach to being able to identify subjects with MGUS and other monoclonal gammopathies.

### Statements of Invention

1. A method of identifying or monitoring a plasma cell associated disease, comprising purifying immunoglobulin free light chains (FLCs) from a sample from a subject with anti-FLC specific antibodies or fragments thereof and subjecting the purified sample to a mass spectrometry technique to identify the presence of one or more peaks corresponding to one or more monoclonal FLCs in the sample.
2. A method according to statement 1, wherein the anti-FLC specific antibodies are a mixture of anti-kappa FLC specific and anti-lambda FLC specific antibodies or fragments thereof.
3. A method of identifying or monitoring a plasma cell associated disease, comprising purifying immunoglobulin free light chains (FLCs) from a sample from a subject with anti-FLC specific antibodies or fragments thereof and subjecting the purified sample to a mass spectrometry technique to identify the presence of one or more peaks corresponding to one or more monoclonal FLCs in the sample, wherein the anti-FLC specific antibodies are a mixture of i) anti-kappa FLC specific antibodies and/or anti-kappa FLC specific fragments thereof, and ii) anti-lambda FLC specific antibodies and/or anti-lambda FLC specific fragments thereof.
4. A method according to statements 1 to 3, wherein the anti-FLC specific antibodies or fragments are polyclonal.
5. A method according to statements 1 to 4, wherein the antibody fragments are F(ab')₂ fragments.
6. A method according to statements 1 to 5, wherein the anti-FLC specific antibodies or fragments, comprise one or more non-disulphide cross-links between at least one heavy chain (or fragment) and one light chain (or fragment) of the antibody or fragments thereof.
7. A method according to statements 1 to 6, wherein the mass spectrometry technique is an Orbitrap mass spectrometer, ion trap mass spectrometer, time-of-flight mass spectrometer, triple quadrupole mass spectrometer, or quadrupole mass spectrometer may be used.
8. A method of statement 7, wherein the mass spectrometry techniques is matrix assisted laser desorption ionisation-time-of-flight mass spectrometry (MALDI-TOF)
9. A method according to any preceding statement, additionally comprising purifying total kappa and total lambda light chains with anti-total kappa and total anti-lambda antibodies or fragments and subjecting the purified sample to mass spectrometry, to identify one or more peaks corresponding to one or more monoclonal immunoglobulins.
10. A method according to statement 8, wherein the total lambda and total kappa light chains are co-purified using a mixture of anti-total kappa and anti-total lambda antibodies.
11. A method according to statements 1 to 10, wherein the plasma cell associated disease is selected from intact immunoglobulin, multiple myeloma, light chain multiple myeloma, non-secretory multiple myeloma, AL amyloidosis, light chain deposition disease (LCDD), smouldering multiple myeloma, monoclonal gammopathy of undetermined significance (MGUS), macroglobulinemia, POEMS (polyneuropathy, organomegaly, endocrinopathy, monoclonal gammopathy and skin changes) syndrome and LCDD.
12. A method according to statements 1 to 11, wherein the sample is tear fluid, plasma, serum, saliva, urine, blood or cerebrospinal fluid.
13. A kit comprising anti-kappa free light chain antibodies, fragments thereof; anti-lambda free light chain antibodies thereof; and a mass spectrometry target.
14. A kit according to statement 13, wherein the antibodies are immobilised on the mass spectrometry target.
15. A mass spectrometer, comprising a mass spectrometry target according to statement 14.

## Claims

1. A method of identifying or monitoring a plasma cell associated disease, comprising:
purifying immunoglobulin free light chains (FLCs) from a sample from a subject with:
i) a mixture of anti-kappa FLC specific antibodies and anti-lambda FLC specific antibodies;
ii) a mixture of anti-kappa FLC specific antibodies and anti-lambda FLC specific antibody fragments;
iii) a mixture of anti-kappa FLC specific antibody fragments and anti-lambda FLC specific antibodies; or
iv) a mixture of anti-kappa FLC specific antibody fragments and anti-lambda FLC specific antibody fragments; and
subjecting the purified sample to a mass spectrometry technique to identify the presence of one or more peaks corresponding to one or more monoclonal FLCs in the sample

2. A method according to claim 1, wherein the anti-kappa FLC specific antibodies, the anti-lambda FLC specific antibodies. the anti-kappa FLC specific antibody fragments, or the anti-lambda FLC specific antibody fragments are polyclonal.

3. A method according to claims 1 or 2, wherein the anti-kappa FLC specific antibody fragments, or the anti-lambda FLC specific antibody fragments are F(ab')₂ fragments.

4. A method according to any of claims 1 to 3, wherein the anti-FLC kappa specific antibodies, or the anti-lambda FLC specific antibodies, comprise one or more non-disulphide cross-links between at least one heavy chain and one light chain of the antibody

5. A method according to any of claims 1 to 4, wherein the anti-kappa FLC specific antibody fragments, or the anti-lambda FLC specific antibody fragments comprise one or more non-disulphide cross-links between at least one heavy chain fragment and one light chain fragment of the fragment of the antibody.

6. A method according to any of claims 1 to 5, wherein the mass spectrometry technique is an Orbitrap mass spectrometer, ion trap mass spectrometer, time-of-flight mass spectrometer, triple quadrupole mass spectrometer, or quadrupole mass spectrometer may be used.

7. A method of claim 6, wherein the mass spectrometry techniques is matrix assisted laser desorption ionisation-time-of-flight mass spectrometry (MALDI-TOF)

8. A method according to any preceding claim, additionally comprising:
purifying total kappa and total lambda light chains with:
i) a mixture of anti-kappa specific antibodies and anti-lambda specific antibodies;
ii) a mixture of anti-kappa specific antibodies and anti-lambda specific antibody fragments;
iii) a mixture of anti-kappa specific antibody fragments and anti-lambda specific antibodies; or
iv) a mixture of anti-kappa specific antibody fragments and anti-lambda specific antibody fragments; and
subjecting the purified sample to mass spectrometry, to identify one or more peaks corresponding to one or more monoclonal immunoglobulins.

9. A method according to any of claims 1 to 8, wherein the plasma cell associated disease is selected from intact immunoglobulin, multiple myeloma, light chain multiple myeloma, non-secretory multiple myeloma, AL amyloidosis, light chain deposition disease (LCDD), smouldering multiple myeloma, monoclonal gammopathy of undetermined significance (MGUS), macroglobulinemia, POEMS (polyneuropathy, organomegaly, endocrinopathy, monoclonal gammopathy and skin changes) syndrome and LCDD.

10. A method according to claims any of 1 to 9, wherein the sample is tear fluid, plasma, serum, saliva, urine, blood or cerebrospinal fluid.
